# EUROPEAN PATENT APPLICATION

(11) **EP 2 278 326 A1**
(43) Date of publication of application: **26.01.2011**
(21) Application number: 09009365.9
(22) Date of filing: 20.07.2009
(51) Int. Cl.: G01N 33/04, G01N 25/04, G01N 25/06

(54) **Method for measuring the cryoscopic point of milk and its by-products with thermistor device**

(71) Applicant: Scarpellini, Alba, 50031 Barberino del Mugello, FI (IT); Mugnai, Giancarlo, 50031 Barberino del Mugello, FI (IT)
(72) Inventor: Scarpellini, Alba, 50031 Barberino del Mugello, FI (IT); Mugnai, Giancarlo, 50031 Barberino del Mugello, FI (IT)
(74) Representative: Paolini, Elena

(57) **Abstract**

The method provides the use of a double hydraulic circuit for cooling a test-tube (5) with the milk or its by-products to be tested.

## Description

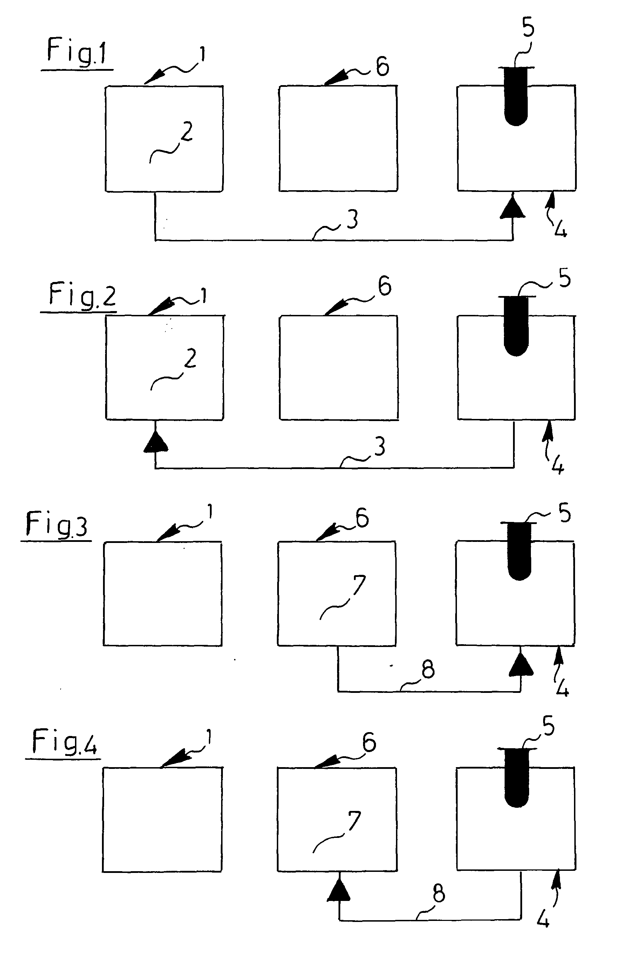

It is known that the measure of the freezing point of the milk and its by products, i.e. of the cryoscopic point, is a method used and standardized to determine the eventual presence of water resulting from errors or failures in the production process or attempted fraud. By means of a thermistor cryoscopic the freezing point of the milk is measured which is close to -0.520°C, where as the presence of water in the analysis sample moves the freezing point measured by the instrument as close to 0°C. The measuring of the deviations from the value of the freezing point of the pure milks permits to value the percentage of extraneous water present in the sample to be tested contained in the test-tube. The measuring is actuated putting the milk or its by-products to be tested in a test-tube with an internal thermistor probe and a stirrer and immerging the test-tube in cooling tank in which the temperature is generally at -7°C. When the temperature of -3°C is reached the milk contained in the test-tube is shaken for inducing the freezing. At the end of this step the temperature measuring is actuated by means of the thermistor probe, determining in this way the cryoscopic point. The remarkable difference between the temperature of the cooling bath which is chosen very low to quickly cool the sample and the measuring temperature of the freezing point around to -0.5°C can be source of errors in the measuring. Further the repeatability of subsequent measuring on the same sample can not be optimum. The subject-matter of present invention provides the use of a double hydraulic cooling circuit that puts fluids in the measuring tank with different temperature on the base of the moment of the measuring process. With the invention so there is the introduction in cooling tank of a fluid at a lower temperature, which rapidly decreases the temperature of the sample in testing, with the temperature of the first fluid to put in cooling tank to be set between -15°C and -5°C, in a second moment is put in the cooling tank a fluid with a temperature close to that of the cryoscopic point, so to create around the test-tube a stable environment and conducive to the precision of the measuring, with the temperature of the second fluid to be set between -5°C and 0°C. The main advantages of this method consist of bigger speed to actuate the measuring of the cryoscopy point, by using a cooling bath with a suitable temperature which brings quickly the sample to be tested to the temperature value necessary, and bigger precision and repeatability of the measuring of the freezing point, due to the microclimate suitable created around the sample in testing. The invented method is articulated in four different steps illustrated in a merely indicative way and not limiting in the scheme of sheet 1. In particular the figure 1 illustrated in schematic view the first step. The figure 2 is schematic view of the second step. Figure 3 is schematic view of the third step. Figure 4 is schematic view of the fourth step. In a preferred embodiment but non exclusive the invented method provides the presence of a tank 1 in which is present a fluid 2 with low temperature, meaning for low temperature a temperature of the fluid 2 between -15°C and -5°C. The fluid 2, by a pipe 3 and fluid movement apparatuses, reaches the tank 4, having the function of measuring bath and into which is present the test-tube 5 with the milk or its by-products to be tested, the thermistor probe and the stirrer. The tank 4 is filled with the fluid 2, starting the cooling of the sample to be tested present into the test-tube 5. The fluid 2 has the task to quickly bring the sample at low temperature for the measuring. At reaching of a prefixed temperature, generally to the reaching of - 3°C, the tank 4 is emptied by the pipe 3 and the auxiliary means, to reach again the tank 1, realizing in this way the second step of the invented method. The third step of the method comprises the use of a tank 6 with a fluid 7 with temperature close to that of measuring, with the task to create the suitable environment for the same measuring. The temperature of the fluid 7 is between -5°C and 0°C. The fluid 7 is brought to the tank 4 with test-tube 5 by a pipe 8 and fluid moving apparatuses. The tank 4 is filled with the fluid 7, so determining the ideal microclimate around the sample in measuring inside the test-tube 5. Ending this third step the tank 4, in the fourth and last step, is emptied by a pipe 8 with the fluid moving apparatuses. The four steps are cyclically repeated for the subsequent measures. The number of tanks 4 with corresponding test-tubes 5, acting as measuring task, is to be varied on the base of the use necessities. The pipes, the fluid moving apparatuses, the distribution devices are to be realized to a person skilled in the art on the base of the invented concept described in the invention. The described method is possible of a lot of changing and variations all part of the present inventive concept. Moreover, all the technical details are to be changed with other equivalent ones.

## Claims

1. Method to measuring the cryoscopic point of milk and its by-products with thermistor device **characterized in that** it provides the use of a double hydraulic circuit for cooling a test-tube (5) with the milk or its by-products to be tested.

2. Method to measuring the cryoscopic point of milk and its by-products with thermistor device, as to the previous claim, **characterized in that** it provides four different steps.

3. Method to measuring the cryoscopic point of milk and its by-products with thermistor device, as to the previous claims, **characterized in that** to have a tank (1) in which is present a fluid (2) with temperature between -15°C and -5°C sent, by a pipe (3) and fluid moving apparatuses, to a tank (4) with task of measuring bath where are present the test-tube (5) with the milk or its by-products to be tested, a thermistor probe and a stirrer.

4. Method to measuring the cryoscopic point of milk and its by-products with thermistor device, as to the previous claims, **characterized in that** at the reaching of the sample to be tested of a temperature close to -3°C, the tank (4) is emptied by a pipe (3) and the auxiliary means to reach again the other tank (1).

5. Method to measuring the cryoscopic point of milk and its by-products with thermistor device, as to the previous claims, **characterized in that** it provides a tank (6) with a fluid (7) having temperature between -5°C and 0°C brought to the tank (4) with test-tube (5) by a pipe (8) and fluid moving apparatuses.

6. Method to measuring the cryoscopic point of milk and its by-products with thermistor device, as to the previous claims, **characterized in that** the tank (4) is emptied of the fluid (7) by a pipe (8) with the fluid moving apparatuses.

7. Method to measuring the cryoscopic point of milk and its by-products with thermistor device, as to the previous claims, **characterized in that** the four steps can be cyclically repeated for the subsequent measures.

8. Method to measuring the cryoscopy point of milk and its by- products with thermistor device, as to the previous claims, **characterized in that** the number of tanks (4) with corresponding test-tubes (5) can be changed on the base of use necessities.
